(19) Europäisches Patentamt
European Patent Office
Office européen des brevets

(11) Veröffentlichungsnummer : **0 157 225**
**B1**

(12) **EUROPÄISCHE PATENTSCHRIFT**

(45) Veröffentlichungstag der Patentschrift : **20.01.88**

(21) Anmeldenummer : **85102693.0**

(22) Anmeldetag : **09.03.85**

(51) Int. Cl.⁴ : **C 07 D277/68**, C 07 D263/58, C 07 D235/26// A01N43/78, A01N43/76, A01N43/52

# (54) Verfahren zur Herstellung von Benzimidazolyl,-Benzoxazolyl- und Benzthiazolyloxyphenoxypropionsäurederivaten.

(30) Priorität : **14.03.84 DE 3409201**
**28.07.84 DE 3427953**

(43) Veröffentlichungstag der Anmeldung : **09.10.85 Patentblatt 85/41**

(45) Bekanntmachung des Hinweises auf die Patenterteilung : **20.01.88 Patentblatt 88/03**

(84) Benannte Vertragsstaaten : **BE CH DE FR GB IT LI NL**

(56) Entgegenhaltungen :
**EP-A- 0 062 905**
**CH-A- 635 326**
**DE-A- 2 640 730**
**DE-A- 3 027 483**
**DE-A- 3 236 730**

(73) Patentinhaber : **CASSELLA Aktiengesellschaft**
**Hanauer Landstrasse 526**
**D-6000 Frankfurt am Main 61 (DE)**

(72) Erfinder : **Kühlein, Klaus, Dr.**
**Fasanenstrasse 41**
**D-6233 Kelkheim/Taunus (DE)**
Erfinder : **Becherer, Johannes, Dr.**
**Weidenseestrasse 8**
**D-6457 Maintal-2 (DE)**
Erfinder : **Kussmaul, Ulrich, Dr.**
**Tannenweg 39a**
**D-6367 Karben 1 (DE)**

(74) Vertreter : **Urbach, Hans-Georg, Dr. et al**
**Hanauer Landstrasse 526**
**D-6000 Frankfurt am Main 61 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 157 225 B1

**Beschreibung**

Die vorliegende Erfindung betrifft ein Verfahren zur Herstellung von Benzimidazolyl-, Benzoxazolyl- und Benzthiazolyloxyphenoxypropionsäurederivaten der Formel I

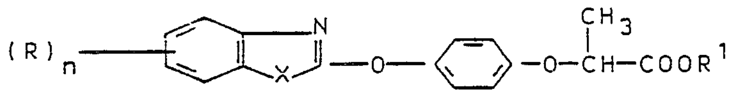

(I)

worin

X Sauerstoff oder Schwefel oder eine Gruppe der Formel = N-Alkyl ($C_1$-$C_4$),

R Halogen oder $CF_3$,

n die Ziffern 0 bis 3 und

$R^1$ ($C_1$-$C_{12}$) Alkyl, das durch ($C_1$-$C_6$) Alkoxy ; ($C_1$-$C_6$) Alkylthio ; ($C_1$-$C_6$) Alkoxy-($C_2$-$C_4$)-alkoxy oder Methoxy-ethoxyethoxy substituiert sein kann ; $C_5$- oder $C_6$-Cyclo-alkyl ; oder ($C_1$-$C_4$) Alkoxycarbonyl-($C_1$ oder $C_2$)-alkyl bedeuten.

Verbindungen der Formel I sind z. B. aus der DE-OS 26 40 730 und der US-Patentschrift 4, 130, 413 bekannt und stellen wertvolle Herbizide dar.

Gemäß der U.S.-Patentschrift 4, 130, 413 erhält man die Verbindungen der Formel I beispielsweise dadurch, daß man Verbindungen der Formel II

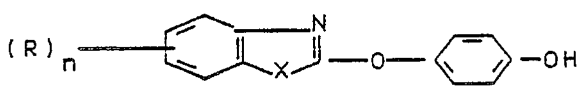

(II)

in der X, R, n und $R^1$ die oben angegebene Bedeutung haben, mit Verbindungen der Formel III

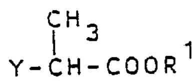

(III)

in der Y Halogen oder eine Sulfonsäureester-Gruppe ist und $R^1$ die oben angeführte Bedeutung hat, in einem inerten aprotischen Lösungsmittel bei erhöhter Temperatur in Gegenwart einer anorganischen oder organischen Base umsetzt.

Dieses Verfahren gemäß dem Stand der Technik hat den gravierenden Nachteil, daß es nur bei Einsatz der 2-Brompropionsäureester der Formel III, worin Y = Br ist, der 2-Jodpropionsäureester der Formel III (Y = J) oder der 2-Tosyloxypropionsäureester der Formel III (Y = Tosyl-O-) zum Endprodukt der Formel I in hoher Ausbeute und in der bei Herbiziden geforderten hohen Reinheit führt. Das Erfordernis, die genannten, in technischem Ausmaß nicht verfügbaren Ausgangsmaterialien der Formel III einsetzen zu müssen, verteuert das Verfahren derartig, daß es bei Einsatz der Endprodukte als Herbizide nicht mehr tragbar ist. Der Einsatz der technisch verfügbaren und billigen Chlorpropionsäureester führt jedoch zu Produkten ungenügender Reinheit in schlechten Ausbeuten, wobei als zusätzlicher Nachteil lange Reaktionszeiten erforderlich sind.

Ein eingehendere Untersuchung der diesem Verfahren zugrunde liegenden Reaktion hat ergeben, daß bei Einsatz der Chlorpropionsäureester als Reaktionskomponente III neben der gewünschten Umsetzung gemäß dem oben angegebenen Schema noch mehrere Nebenreaktionen ablaufen, die zu unerwünschten Nebenprodukten führen und die Ausbeute an Zielprodukt drastisch herabsetzen.

Folgende Nebenreaktionen wurden beobachtet :

a) konkurrierende C-Alkylierung ;

b) Hydrolyse vorhandener Esterfunktionen durch Folgeprodukte der eingesetzten Basen (z. B. $H_2O$ aus $KHCO_3$) ;

c) Umesterungsreaktionen : aus aliphatischen Estern werden Phenol- bzw. Hydrochinonester ;

d) Spaltung der Heteroaryletherbindung in den Ausgangsprodukten der Formel II ;

e) Spaltung der Heteroaryletherbindung in den Endprodukten der Formel I,

f) Bildung von symmetrischen Ethern der Formel

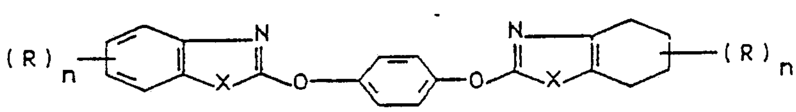

g) Bildung von symmetrischen Ethern der Formel

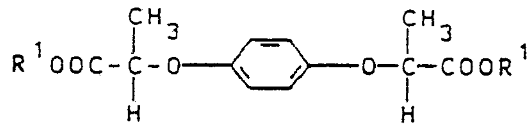

h) Hydrolyse der Ausgangsverbindungen der Formel III zu Milchsäurederivaten ;

i) Spaltung der Heterocyclen in den Ausgangsprodukten der Formel II zu ringoffenen Folgeprodukten ;

k) Spaltung der Heterocyclen in den Endprodukten der Formel I zu ringoffenen Folgeprodukten.

Bei dieser großen Anzahl von nachgewiesenen Nebenreaktionen wird es verständlich, daß die Umsetzungsprodukte nicht mehr einheitlich sein können. Demzufolge ist das Zielprodukt aus dem Reaktionsgemisch nur durch umständliche und in technischem Ausmaß kaum durchführbare Reinigungsoperationen zu erhalten. Zu dem wegen der Nebenreaktion an sich schon geringen Umsetzungsanteil der Ausgangsprodukte zum Zielprodukt kommen noch die unvermeidlichen Reinigungsverluste, so daß im Endeffekt nur Ausbeuten zwischen 40 und 50 % der Theorie an reinem Zielprodukt erhalten werden.

Es wurde nun gefunden, daß sich überraschenderweise alle oben genannten Nebenreaktionen weitestgehend zurückdrängen und damit die Nachteile des Standes der Technik vermeiden lassen, wenn man den Reaktionsgemischen bestehend aus Hydrochinonmonoethern der Formel II und 2-Chlorpropionsäureestern der Formel III (Y = Cl) katalytische Mengen Metalljodide oder der entsprechenden 2-Jodpropionsäureester zusetzt. Dabei können prinzipiell alle möglichen Jodide eingesetzt werden, zweckmäßigerweise wird man jedoch die Jodide der Alkalimetalle und Erdalkalimetalle, wie z. B. Natriumjodid oder Kaliumjodid, aufgrund ihrer leichten Verfügbarkeit bevorzugen.

Es wurde ferner gefunden, daß man bei dem erfindungsgemäßen Verfahren die Ausgangsverbindungen der Formel II nicht in isolierter Form einzusetzen braucht, sondern daß man das Verfahren zur Herstellung der Verbindungen der Formel I vorteilhafterweise auch so durchführen kann, daß zunächst eine Verbindung der Formel IV

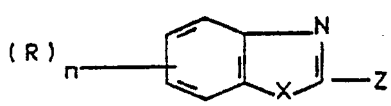

(IV)

in der R und n die bereits vorstehend angegebenen Bedeutungen besitzen und Z Halogen, vorzugsweise Chlor oder Brom, oder eine andere Austrittsgruppe bedeutet, mit Hydrochinon in Gegenwart einer Base und dann ohne Isolierung mit einem 2-Chlorpropionsäureester der Formel III mit Y = Chlor in Gegenwart katalytischer Mengen Metalliodid und/oder 2-Iodpropionsäureester der Formel III mit Y = Iod umgesetzt wird oder daß besonders vorteilhafterweise eine Verbindung der Formel IV mit Hydrochinon und mit einem 2-Chlorpropionsäureester der Formel III mit Y = Chlor in Gegenwart einer Base und in Gegenwart katalytischer Mengen Metalliodid und/oder 2-Iodpropionsäureester der Formel III mit Y = Iod umgesetzt wird.

X bedeutet vorzugsweise Sauerstoff, Schwefel oder die Gruppe = $N-CH_3$. R bedeutet vorzugsweise Chlor, Brom oder $CF_3$ n bedeutet vorzugsweise 0 oder 1 und $R^1$ bedeutet vorzugsweise $(C_1-C_4)$ Alkyl, Cyclohexyl und durch Methoxysubstituiertes $(C_1-C_4)$ Alkyl.

Diese besonders vorteilhafte Variante des erfindungsgemäßen Verfahrens wird in einem Reaktionsgefäß ohne Isolierung oder Reinigung der Zwischenprodukte durchgeführt. Die Molverhältnisse zwischen den Ausgangsverbindungen der Formel IV, Hydrochinon und dem 2-Chlorpropionsäureester der Formel III mit Y = Chlor können in weiten Grenzen variiert werden. Günstige Molverhältnisse zwischen den Verbindungen der Formel IV : Hydrochinon : 2-Chlorpropionsäureester der Formel III mit Y = Chlor sind = 1 : (1 bis 1,5) : (1,3 bis 5), vorzugsweise = 1 : (1 bis 1,1) : (1,3 bis 2,5). Aus den angegebenen Molverhältnissen geht hervor, daß Hydrochinon und der 2-Chlorpropionsäureester der Formel III mit Y = Chlor im Überschuß eingesetzt werden. Noch größere Überschüsse an der Verbindung der Formel III und/oder an Hydrochinon einzusetzen, ist zwar möglich, aber nicht mehr zweckmäßig. Ein Überschuß an 2-Chlorpropionsäureester der Formel III mit Y = Chlor kann leicht, z. B. durch Destillation, zurückgewonnen werden.

Der Einsatz der Ausgangsverbindungen der Formel IV im Überschuß ist zwar prinzipiell auch möglich, die Regenerierung der Verbindungen IV oder deren Abtrennung von den Produkten I ist jedoch in der Regel technisch aufwendiger. Außerdem werden schlechtere Ausbeuten und Reinheiten der Endprodukte der Formel I erzielt.

Die Menge der eingesetzten Jodide bzw. des 2-Jodpropionsäureesters kann in weiten Grenzen variiert werden. In der Regel erfolgt ein Zusatz von 0,1 bis 25 Mol-%, vorzugsweise von 1 bis 20 Mol-%, bezogen auf den Einsatz der Verbindungen der Formel II bzw. IV. Naturgemäß kann auch noch mehr Jodid bzw. Jodpropionsäureester eingesetzt werden, was jedoch in der Regel nicht erforderlich ist.

Selbstverständlich können auch Mischungen der angegebenen, als Katalysator wirkenden Jodverbindungen eingesetzt werden.

Es wird bevorzugt, als Katalysator direkt Metalljodide einzusetzen, weil 2-Jodpropionsäureester zunächst hergestellt werden müssen, was naturgemäß einen erhöhten technischen Aufwand bedeutet, der in der Regel gegenüber dem Jodid-Zusatz keine zusätzlichen Vorteile bringt.

Das Molverhältnis der Ausgangsverbindungen der Formeln II und III kann in weiten Grenzen variiert werden. Günstig ist ein Molverhältnis der Verbindungen II : III von 1 : 1 bis 1 : 5, vorzugsweise 1 : 1,3 bis 1 : 2,5. Noch größere Überschüsse an III sind zwar möglich aber nicht mehr zweckmäßig. Überschüssige Verbindung III kann leicht z. B. durch Destillation regeneriert werden. Der Einsatz der Ausgangsverbindungen der Formel II im Überschuß ist zwar prinzipiell auch möglich, die Regenerierung der Verbindungen II oder deren Abtrennung von den Produkten I ist jedoch in der Regel technisch aufwendiger. Außerdem werden schlechtere Ausbeuten und Reinheiten der Endprodukte der Formel I erzielt.

Wird das erfindungsgemäße Verfahren mit überschüssigem Chlorpropionsäureester der Formel III (Y=Cl) durchgeführt und wird nach erfolgter Reaktion der überschüssige Chlorpropionsäureester abdestilliert und in den Folgeansätzen wieder eingesetzt, so läßt sich die erforderliche Menge an Jodidsalzen in den Folgeansätzen drastisch senken. In manchen Fällen kann sogar ganz darauf verzichtet werden, da dann die Katalyse durch 2-Jodpropionsäureester der Formel III (Y=J) erfolgt, der in dem aus dem vorhergehenden Ansatz regenerierten überschüssigen Chlorpropionsäureester enthalten ist.

Wie üblich ist es zweckmäßig, die Umsetzung der Ausgangsstoffe in einem inerten organischen Lösungsmittel durchzuführen. Vorzugsweise werden aprotisch polare Lösungsmittel eingesetzt. So kommen z. B. Ketone wie Aceton, Methylethylketon oder Methylisobutylketon ; Säureamide wie Dimethylformamid, Dimethylacetamid, Diethylacetamid, N-Methylpyrrolidon, Hexamethylphosphorsäuretrisamid, ferner Dimethylsulfoxid zum Einsatz. Außerdem können auch Nitrile wie Acetonitril oder Propionitril eingesetzt werden. Selbstverständlich sind auch Lösungsmittelgemische aus Vertretern aus den erwähnten Lösungsmittelklassen, die in weiten Grenzen variiert werden können, möglich. Auch Mischungen der erwähnten Lösungsmittel mit anderen inerten Lösungsmitteln, wie z. B. aromatischen Kohlenwasserstoffen wie Toluol und Xylol, sind möglich.

Die eingesetzten Lösungsmittel müssen technisch trocken sein, um eine Verseifung der Esterfunktionen der Verbindungen der Formeln I und III zu vermeiden. Auch eventuell gebildetes Reaktionswasser ist durch bekannte Maßnahmen, wie z. B. Destillation, Destillation mit Lösungsmitteln, die mit Wasser Aceotrope bilden, oder Trocknen mit Trokkenmitteln, zu entfernen.

Zusätzlich werden den Reaktionsgemischen Basen in an sich bekannter Weise zugesetzt. Als basische Verbindungen eignen sich die üblichen organischen und anorganischen Basen, wie z. B. tertiäre Amine wie Pyridin, Triethylamin, Alkoholate, wie Na- oder K-Methylat, -Ethylat, oder -Butylat, insbesondere jedoch anorganische Basen wie Kalium- oder Natriumhydroxid, Magnesiumoxid, Zinkoxid, Calciumoxid oder die entsprechenden Carbonate, insbesondere Alkalicarbonate, wie z. B. Natrium- oder Kaliumcarbonat. Auch Hydride wie z. B. Natriumhydrid sowie Gemische verschiedener Basen können eingesetzt werden.

Die Basen werden in an sich bekannter Weise in mindestens stöchiometrischen Mengen, vorzugsweise jedoch in einem Überschuß von 10 bis 100 %, eingesetzt. Größere Überschüsse sind möglich, bieten jedoch keinen Vorteil und führen zu einer erhöhten Salzbelastung.

Die Reaktionstemperaturen liegen zwischen 50 und 250 °C, vorzugsweise zwischen 80 und 150 °C, insbesondere zwischen 80 °C und dem Siedepunkt des Lösungsmittels.

Arbeitet man nach der bevorzugten Ausführungsform des erfindungsgemäßen Verfahrens ausgehend von Verbindungen der Formel IV, so wird entweder zuerst eine Verbindung der Formel IV mit Hydrochinon und dann mit einem 2-Chlorpropionsäureester der Formel III mit Y = Chlor umgesetzt, oder es werden die drei Komponenten der Formel IV, Hydrochinon und der 2-Chlorpropionsäureester der Formel III mit Y = Chlor von Anfang an zusammen zur Reaktion gebracht. Es kann gelegentlich auch Vorteile bieten, einzelne Ausgangskomponenten während der laufenden Umsetzung ein-oder mehrmals nachzusetzen. Die Base wird von Anfang an mit eingesetzt. Es kann zweckmäßig sein, zuerst das Hydrochinon durch Umsetzung mit der Base in das Hydrochinonsalz zu überführen. Wesentlich ist, daß mit der Zugabe des 2-Chlorpropionsäureesters der Formel III mit Y = Chlor auch der Katalysator (Iodid und/oder 2-Iodpropionsäureester der Formel III mit Y = Iod) zugegeben wird.

Das erfindungsgemäße Verfahren wird zweckmäßigerweise in einer Inertgasatmosphäre, z. B. einer Stickstoffatmosphäre, ausgeführt, um unerwünschte Oxidationsprozesse zu vermeiden. Zur Vermeidung von derartigen Oxidationsprozessen ist es auch möglich, den Ansätzen Reduktionsmittel, wie z. B. Natriumsulfit, zuzusetzen.

Die Umsetzung der Ausgangsmaterialien nach dem erfindungsgemäßen Verfahren verläuft sehr einheitlich und mit überraschend großer Geschwindigkeit.

Selbstverständlich können die Reaktionen auch bei anderen Drücken als dem Atmosphärendruck durchgeführt werden. Die daraus resultierenden sehr kurzen Reaktionszeiten, die je nach Ausgangsmaterial und benutzten Verfahrensbedingungen in vielen Fällen unter ca. 10 Minuten liegen, erlauben eine Durchführung des erfindungsgemäßen Verfahrens sowohl in kontinuierlicher als auch diskontinuierlicher Weise.

Die Aufarbeitung der Reaktionsgemische ist einfach und an sich bekannt. Nach Abtrennung der

Salze werden Lösungsmittel und evtl. überschüssige Reaktanten abdestilliert und gegebenenfalls nach Trocknung wieder eingesetzt. Der Rückstand wird, falls es zur weiteren Reinigung erforderlich sein sollte, gegebenenfalls nach Aufnahme in einem mit Wasser nicht mischbaren Lösungsmittel nochmals mit Wasser gewaschen oder aus einem geeigneten Lösungsmittel oder Lösungsmittelgemisch kristallisiert.

Die Ausgangsverbindungen der Formel II und ihre Herstellung sind aus der Europäischen Patentschrift Nr. 924 bekannt.

Gegenüber dem bekannten liefert das erfindungsgemäße Verfahren die Verbindungen der Formel I in sehr erheblich verbesserter Ausbeute, sehr guter Reinheit und unter Verwendung preisgünstiger, gut zugänglicher Ausgangsmaterialien.

Bei der Durchführung der bevorzugten Ausführungsform, ausgehend von Verbindungen der Formel IV kann überraschenderweise auch noch auf die aufwendige und kostenintensive Herstellung von Zwischenprodukten verzichtet werden, und auch hier verläuft die Umsetzung der Ausgangsmaterialien sehr einheitlich und mit überraschend großer Geschwindigkeit. In vielen Fällen, insbesondere, wenn die drei Reaktionskomponenten Hydrochinon, Verbindung der Formel IV und der 2-Chlorpropionsäureester der Formel III mit Y = Chlor von Anfang an zusammen zur Reaktion gebracht werden, beträgt die Reaktionszeit weniger als 1 Stunde.

Daß insbesondere diese vorteilhafte Verfahrensvariante des erfindungsgemäßen Verfahrens zum Erfolg führt, ist aus mehreren Gründen als äußerst überraschend zu bezeichnen. So kann Hydrochinon bereits mit jedem einzelnen der beiden weiteren Ausgangskomponenten in verschiedener Weise reagieren, so daß insgesamt eine Vielzahl größtenteils unerwünschter Reaktionsmöglichkeiten besteht. Beispielsweise wird beobachtet und ist dem Fachmann bekannt, daß Hydrochinon mit 2-Chlorpropionsäureestern der Formel III mit Y = Chlor zu unerwünschten symmetrischen Ethern der oben angegebenen Struktur führt.

Auch die Reaktion von Hydrochinon mit Verbindungen der Formel IV verläuft nicht ganz einheitlich, vielmehr entstehen auch hier als Nebenprodukt die entsprechenden Bis-ether der ebenfalls oben genannten Struktur.

Schließlich kann beispielsweise auch die Alkylierung der Verbindungen der allgemeinen Formel II zu zehn unerwünschten Nebenreaktionen führen, wie sie im einzelnen oben angeführt sind. Vor dem Hintergrund dieser Vielzahl möglicher Konkurrenzreaktionen muß es als sehr überraschend angesehen werden, daß die erfindungsgemäße Mehrkomponentenreaktion in Reaktionszeiten von oft weniger als einer Stunde zu den gewünschten Endprodukten in hoher Ausbeute und Reinheit führt.

Die folgenden Ausführungsbeispiele veranschaulichen die Durchführung des erfindungsgemäßen Verfahrens.

## Beispiel 1

In einem 0,3-Liter-Rührautoklaven werden folgende Ausgangsverbindungen 12 Stunden lang auf 90-95 °C erhitzt, wobei ein maximaler Druck von 2,7 bar entsteht :

160 ml technisch trockenes Aceton
27,6 g Kaliumkarbonat
27,2 g 2-Chlorpropionsäureethylester
2,2 g Natriumjodid und
27,6 g 4(6′-Chlorbenzothiazolyl-2′-oxy) phenol.

Zur Aufarbeitung wird das Reaktionsgemisch von dem ausgefallenen Salzen abgesaugt und aus dem Filtrat das Aceton sowie überschüssiger Halogenpropionsäureethylester zunächst unter Atmosphärendruck und dann im Wasserstrahlvakuum abdestilliert. Der Rückstand wird in Xylol aufgenommen und die Xylolphase nochmals mit Wasser gewaschen. Die Xylolphase enthält das gewünschte Produkt und kann in dieser Form weiterverarbeitet werden. Zur Charakterisierung wird Xylol im Wasserstrahlvakuum abdestilliert. Als Rückstand erhält man

36,2 g 4(6′-Chlorbenzothiazolyl-2′-oxy) phenoxypropionsäureethylester vom Schmelzpunkt 53 bis 55 °C, der im Dünnschichtchromatogramm keine erkennbaren Verunreinigungen enthält.

Ausbeute : 96 % d. Th.

## Vergleichbeispiel zu Beispiel 1

Es wird analog Beispiel 1 verfahren, jedoch wird dem Reaktionsgemisch kein Natriumjodid zugesetzt. Ein Dünnschichtchromatogramm zeigt, daß kein vollständiger Umsatz erfolgt und daß sich zahlreiche unerwünschte Nebenprodukte in größerer Menge gebildet haben. Man erhält 32,6 öligen, klebrigen Rückstand, der u. a. mit Hydrochinon-bis-4(6′-chlorbenzothiazolyl-) ether verunreinigt ist und nicht kristallisiert.

Nach wiederholter Behandlung mit wäßrigem Ethanol unter Zusatz von Aktivkohle, oder durch Säulenchromatographie kann aus dem nicht kristallisierenden Rohprodukt das chromatographisch einheitliche Zielprodukt in einer Ausbeute von 15 g entsprechend 39,7 % d. Th. gewonnen werden.

Beispiel 2

Das Aceton-Destillat aus dem Beispiel 1 wird über wasserfreiem Natriumsulfat getrocknet und analysiert. Es besteht im wesentlichen aus Aceton, überschüssigem 2-Chlorpropionsäureethylester und während der Reaktion gemäß Beispiel 1 gebildetem 2-Jodpropionsäureester. Dem getrockneten Filtrat werden wieder

27,6 g 4(6'-Chlorbenzothiazolyl-2'-oxy) phenol
27,6 g Kaliumkarbonat
13,6 g frischer 2-Chlorpropionsäureethylester und nur
0,7 g Natriumjodid

zugesetzt und die Mischung, wie im Beispiel 1 angegeben, 12 Stunden auf 90-95 °C erwärmt und anschließend aufgearbeitet.

Die Ausbeute beträgt 36,5 g 4(6'-Chlorbenzothiazolyl-2'-oxy) phenoxypropionsäureethylester vom Schmelzpunkt 53-55 °C.

Ausbeute : 96 %, dünnschichtchromatographisch rein.

Beispiel 3

Die Ausgangsstoffe gemäß Beispiel 1 werden in 80 ml trockenem Dimethylformamid (DMF) statt Aceton 10 Minuten lang bei 142-144 °C im offenen Gefäß gerührt. Die Aufarbeitung gemäß Beispiel 1 ergibt

35,9 g Produkt vom Schmelzpunkt 53,5 bis 55 °C, entsprechend 95,2 % d. Th.

Wird analog Beispiel 2 verfahren, wobei das Destillat aus dem ersten Ansatz, bestehend aus DMF, 2-Chlorpropionsäureester und 2-Jodpropionsäureester wieder eingesetzt wird, so erhält man

36,7 g Produkt vom Schmelzpunkt 53 bis 55 °C, entsprechend einer Ausbeute von 97,3 % d. Th.

Auch der Einsatz von nur 1 Mol-% Natriumjodid bringt keine Nachteile.

Anstelle von DMF kann im obigen Beispiel auch die gleiche Menge Dimethylsulfoxid eingesetzt werden. Man erhält in diesem Fall eine Ausbeute von 95,8 % d. Th. an chromatographisch reiner Substanz.

Beispiel 4

Analog Beispiel 1 wird im Rührautoklaven gearbeitet ; wobei jedoch als Lösungsmittel Acetonitril statt Aceton eingesetzt wird. Die Reaktionszeit beträgt 14 Stunden bei 90-95 °C ; der maximale Druck 1,2 bar. Man erhält

35,5 g Produkt, entsprechend 94,1 % d. Th. mit einem Schmelzpunkt von 51 bis 53 °C.

Beispiel 5

In einem 0,3-Liter-Euzonit-Rührautoklaven werden
150 ml technisch trockenes Methylethylketon
21,0 g Kaliumkarbonat
29,0 g 2-Chlorpropionsäureethylester
2,3 g Natriumjodid und
26,2 g 4(6'-Chlorbenzoxazolyl-2'-oxy) phenol

10 Stunden auf 130 °C Innentemperatur erhitzt. Der maximale Druck ist 5 bar. Man kühlt ab, saugt von den Salzen ab, wäscht mit etwas Methylethylketon nach und destilliert das Lösungsmittel zusammen mit dem überschüssigen 2-Chlorpropionsäureethylester und gebildeten 2-Jodpropionsäureethylester zunächst bei Atmosphärendruck und dann im Wasserstrahlvakuum ab. Der in der Hitze flüssige Destillationsrückstand wird aus 60 %igem wäßrigen Ethanol kristallisiert. Man erhält

29 g 4(6'-Chlorbenzoxazolyl-2'-oxy) phenoxipropionsäureethylester vom Schmelzpunkt 82-84 °C, welcher keine dünnschichtchromatographisch erkennbare Verunreinigungen enthält.

Ausbeute : 80 % d. Th.

Vergleichsbeispiel zu Beispiel 5

Verfährt man wie im Beispiel 5, läßt jedoch das Natriumjodid weg, so ist die Reaktion nach 10 Stunden nur etwa zur Hälfte abgelaufen. Nach 20 Stunden ist dünnschichtchromatographisch immer noch Ausgangsprodukt zu sehen. Aufarbeitung wie im Beispiel 5 ergibt eine Ausbeute von nur 55 %.

Beispiel 6

In einem 250-ml-Kolben mit Rührer, Rückflußkühler, Thermometer und Gaseinleitung werden unter Stickstoff

150 ml technisch trockenes M-Methylpyrrolidon

21,0 g Kaliumkarbonat
29,0 g 2-Chlorpropionsäureethylester
2,3 g Natriumjodid und
26,2 g 4(6'-Chlorbenzoxazolyl-2'-oxy) phenol
10 Minuten auf 140 °C erhitzt. Dann wird abgekühlt, von den Salzen abgesaugt, mit N-Methylpyrrolidon nachgewaschen, das Lösungsmittel zusammen mit überschüssigem 2-Chlorpropionsäureethylester und gebildetem 2-Jodpropionsäureethylester im Vakuum abdestilliert und der flüssige heiße Destillationsrückstand aus 60 %igem wäßrigen Ethanol kristallisiert. Man erhält

30,8 g 4(6'-Chlorbenzoxazolyl-2'-oxy) phenoxypropionsäureethylester vom Schmelzpunkt 83-84 °C, welcher keine dünnschichtchromatographisch nachweisbare Verunreinigungen enthält.

Ausbeute : 85 % d. Th.

## Beispiel 7

Arbeitet man wie im Beispiel 6, setzt jedoch Dimethylsulfoxid statt N-Methylpyrrolidon als Lösungsmittel und nur 0,8 g Natriumjodid ein, so erhält man den 4(6'-Chlorbenzoxazolyl-2'-oxy) phenoxypropionsäureethylester in 82 % Ausbeute und vergleichbarer Qualität.

## Beispiel 8

1 mol 2,6-Dichlorbenzoxazol, 0,8 mol Hydrochinon, 2 mol 2-Chlorpropionsäureethylester, 3 mol Kaliumcarbonat, 0,15 mol Natriumiodid, 0,1 mol wasserfreies Natriumsulfit und 300 ml Toluol werden in 900 ml N-Methylpyrrolidon bei Raumtemperatur zusammengegeben und unter gutem Rühren in einem vorgeheizten Ölbad im Verlauf von 30 bis maximal 40 min auf eine Innentemperatur von etwa 145 °C hochgeheizt. Bei Innentemperatur 135 °C werden 0,2 mol, bei Innentemperatur 140 °C weitere 0,1 mol Hydrochinon zugesetzt. Ab Innentemperatur 100 °C tritt $CO_2$-Entwicklung auf, ab etwa 105 °C destilliert ein Toluol-Wasser-Gemisch ab. Sobald der Ansatz nach ca. 35 min 145 °C Innentemperatur erreicht hat, wird das Ölbad durch ein Eiswasserbad ersetzt, der Ansatz auf Raumtemperatur abgekühlt, vom Salz abgesaugt, mit 300 ml N-Methylpyrrolidon und dann mit 1 l Ethanol gewaschen. Die N-Methylpyrrolidonfiltrate werden bei einem Druck von 0,013 mbar bis zu einer Innentemperatur von 110 °C destilliert, der Sumpf mit dem oben angefallenen Ethanolfiltrat versetzt und auf Zimmertemperatur abgekühlt, wobei das Produkt größtenteils auskristallisiert. Zur Vervollständigung der Fällung werden 600 ml Wasser langsam und unter gutem Rühren zugetropft, dann auf 10 °C abgekühlt, abgesaugt und mit 500 ml 65 % igem wäßrigen Ethanol nachgewaschen. Man erhält nach dem Trocknen 318 g 4(6'-Chlorbenzoxazolyl-2'-oxy) phenoxypropionsäureethylester vom Schmelzpunkt 82 bis 84 °C, entsprechend einer Ausbeute von 87,8 % d. Th.

## Beispiel 9

In einem Gemisch von 160 ml N-Methylpyrrolidon und 40 ml Toluol werden zunächst 0,206 mol Hydrochinon und 0,6 mol Kaliumcarbonat am Wasserabscheider bis zur Entfernung des Reaktionswassers gekocht, dann werden 0,2 mol 2,6-Dichlorbenzothiazol zugegeben und weiter Wasser ausgekreist. Schließlich werden bei 140 °C 1,7 g Kaliumiodid und 0,4 mol 2-Chlorpropionsäureethylester zugesetzt und nach 10 min Rühren bei 140 °C auf Raumtemperatur abgekühlt. Der Salzrückstand wird abfiltriert und mit wenig N-Methylpyrrolidon nachgewaschen. Überschüssiger 2-Chlorpropionsäureethylester und Lösungsmittel werden durch Destillation bei einem Druck von ca. 15 mbar zurückgewonnen. Der Destillationsrückstand wird in Xylol aufgenommen und mit 0,5 %iger Natronlauge und dann Wasser gewaschen. Man erhält nach destillativer Entfernung des Xylols 71,8 g 4(6'-Chlorbenzothiazol-2'-oxy) phenoxypropionsäureethylester vom Schmelzpunkt 50 bis 52 °C, entsprechend einer Ausbeute von 95,2 % d. Th.

In Analogie zu den obigen Beispielen lassen sich auch die Verbindungen der folgenden Tabelle erhalten.

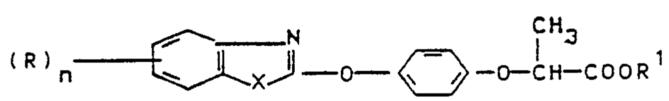

(I)

(Siehe Tabelle Seite 8 f.)

Tabelle

(R)n R1 X Ausbeute (%) d.Th. Fp:

| $(R)_n$ | $R^1$ | X | Ausbeute (%) d.Th. | Fp: |
|---|---|---|---|---|
| H | $-CH_3$ | S | 96 | 75-76°C |
| 5-Cl | $-C_2H_5$ | S | 94 | 92-94°C |
| $5-CF_3$ | $-C_2H_5$ | S | 89 | 65-67°C |
| 6-Br | $-C_2H_5$ | S | 94 | 53-54°C |
| 5-Cl | $-CH_3$ | O | 88 | 76-78°C |
| 5-Cl | $-CH_2CH_2-O-CH_3$ | O | 83 | 103-106°C |
| 6-Cl | -isobutyl | O | 78 | 50-52°C |
| 6-Cl | -cyclohexyl | O | 85 | 92-93°C |
| H | $-C_2H_5$ | $N-CH_3$ | 81 | 85-87°C |

**Patentansprüche**

1. Verfahren zur Herstellung von Benzimidazolyl-, Benzoxazolyl- und Benzthiazolyloxyphenoxypropionsäurederivaten der Formel I

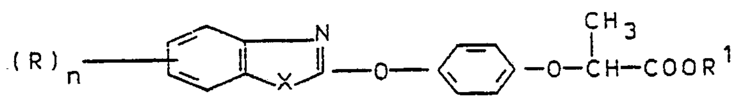

(I)

worin

X Sauerstoff oder Schwefel oder eine Gruppe der Formel = N-Alkyl ($C_1$-$C_4$),
R Halogen oder $CF_3$,
n die Ziffern 0 bis 3 und
$R^1$ ($C_1$-$C_{12}$) Alkyl, das durch ($C_1$-$C_6$) Alkoxy ; ($C_1$-$C_6$) Alkylthio ; ($C_1$-$C_6$) Alkoxy-($C_2$-$C_4$)-alkoxy ; oder Methoxyethoxyethoxy substituiert sein kann ; $C_5$- oder $C_6$-Cycloalkyl ; oder ($C_1$-$C_4$) Alkoxycarbonyl-($C_1$ oder $C_2$)-alkyl, bedeuten,

durch Reaktion von Verbindungen der Formel II

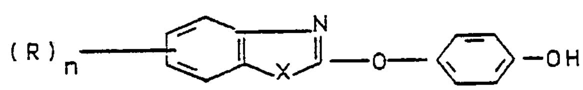

(II)

in der X, R, und n die oben angegebene Bedeutung haben, mit Verbindungen der Formel III

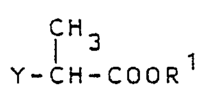

(III)

in der Y Chlor ist und $R^1$ die oben angeführte Bedeutung hat, dadurch gekennzeichnet, daß man den Reaktionsgemischen bei Reaktionstemperaturen von 50 bis 250 °C katalytische Mengen Metalljodide und/oder 2-Jodpropionsäureester der Formel III mit Y = J sowie Basen in mindestens stöchiometrischen Mengen zusetzt.

2. Verfahren gemäß Anspruch 1, dadurch gekennzeichnet, daß eine Verbindung der Formel IV

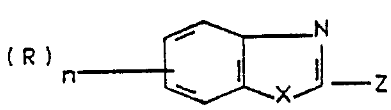

(IV)

in der R und n die vorstehend angegebenen Bedeutungen besitzen und Z halogen oder eine andere Austrittsgruppe bedeutet, mit Hydrochinon in Gegenwart einer Base und dann ohne Isolierung mit einem 2-Chlorpropionsäureester der Formel III

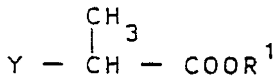

(III)

in der Y Chlor bedeutet und $R^1$ die vorstehend angegebene Bedeutung besitzt, in Gegenwart katalytischer Mengen Metalliodid und/oder 2-Iodpropionsäureester der Formel III mit Y = Iod umgesetzt oder daß eine Verbindung der Formel IV mit Hydrochinon und mit einem 2-Chlorpropionsäureester der Formel III mit Y = Chlor in Gegenwart einer Base und in Gegenwart katalytischer Mengen Metalliodid und/oder 2-Iodpropionsäureester der Formel III mit Y = Iod umgesetzt wird.

3. Verfahren gemäß Anspruch 1, dadurch gekennzeichnet, daß man die Ausgangsmaterialien der Formeln II und III im Molverhältnis von mindestens 1 : 1 bis maximal 1 : 5 zur Reaktion bringt.

4. Verfahren gemäß Anspruch 2, dadurch gekennzeichnet, daß bei der Umsetzung das Molverhältnis Verbindung der Formel IV : Hydrochinon : 2-Chlorpropionsäureester der Formel III mit Y = Chlor = 1 : (1 bis 1,5) : (1,3 bis 5) beträgt.

5. Verfahren gemäß den Ansprüchen 1 bis 4, dadurch gekennzeichnet, daß, bezogen auf die Verbindung der Formel II bzw. IV, man den Reaktionsgemischen 0,1 bis 25 Mol-% Metalliodide und/oder 2-Iodpropionsäureester zusetzt.

6. Verfahren gemäß den Ansprüchen 1 bis 5, dadurch gekennzeichnet, daß man die Reaktion in technisch wasserfreien aprotischen polaren Lösungsmitteln oder in Lösungsmittelgemischen, die technisch wasserfreie aprotisch polare Lösungsmittel enthalten, ausführt und während der Reaktion gegebenenfalls entstehendes Reaktionswasser aus dem Ansatz entfernt.

7. Verfahren gemäß den Ansprüchen 1 bis 6, dadurch gekennzeichnet, daß man das Lösungsmittel und überschüssigen 2-Chlorpropionsäureester der Formel III sowie gebildeten 2-Jodpropionsäureester nach der Umsetzung abdestilliert und das Destillat bei dem folgenden Ansatz wieder einsetzt.

**Claims**

1. Process for the preparation of benzimidazolyl-, benzoxazolyl- and benzthiazolyl-oxyphenoxypropionic acid derivatives of the formula I

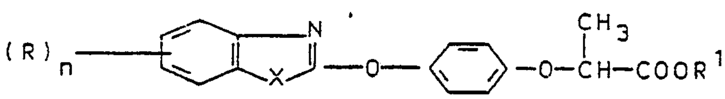

(I)

wherein

X denotes oxygen or sulphur or a group of the formula = N-alkyl ($C_1$-$C_4$),
R denotes halogen or $CF_3$,
n denotes the numbers 0 to 3 and
$R^1$ denotes ($C_1$-$C_{12}$)-alkyl which can be substituted by ($C_1$-$C_6$)-alkoxy, ($C_1$-$C_6$)-alkylthio, ($C_1$-$C_6$)-alkoxy-($C_2$-$C_4$)-alkoxy or methoxyethoxyethoxy; $C_5$-cycloalkyl or $C_6$-cycloalkyl; or ($C_1$-$C_4$)-alkoxycarbonyl-($C_1$ or $C_2$)-alkyl,

by reacting compounds of the formula II

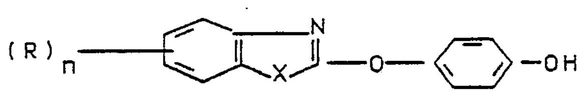

(II)

in which X, R and n have the meaning indicated above, with compounds of the formula III

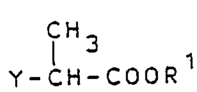

(III)

in which Y is chlorine and $R^1$ has the meaning indicated above, characterised in that catalytic amounts of metal iodide and/or 2-iodopropionic acid esters of the formula III in which Y = I as well as bases are added to the reaction mixtures at temperatures of from 50 to 250 °C in at least stoichiometric amounts.

2. Process according to claim 1, characterised in that a compound of formula IV

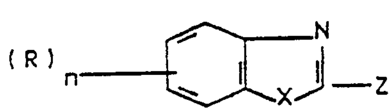

(IV)

wherein R and n have the meanings indicated above and Z is halogen or a different eliminatable

substituent is reacted with hydroquinone in the presence of a base and then, without isolation, with a 2-chloropropionic acid ester of the formula III

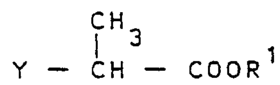 (III)

wherein Y is chlorine and $R^1$ has the meaning indicated above, in the presence of catalytic amounts of metal iodide and/or 2-iodopropionic acid ester of the formula III, Y representing iodine, or that a compound of the formula IV is reacted with hydroquinone or with a 2-chloropropionic acid ester of the formula III, Y denoting chlorine, in the presence of a base and in the presence of catalytic amounts of metal iodide and/or 2-iodopropionic acid ester of the formula III, Y denoting iodine.

3. Process according to claim 1, characterised in that the starting materials of the formulae II and III are reacted in a molar ratio of at least 1 : 1 to not more than 1 : 5.

4. Process according to claim 2, characterised in that in the reaction the molar ratio of compound of formula IV : hydroquinone : 2-chloropropionic acid ester of formula III, Y denoting chlorine, is 1 : (1 to 1.5) : (1.3.to 5).

5. Process according to Claims 1 to 4, characterised in that, relative to the compound of formula II or IV, 0.1 to 25 mol % of metal iodides and/or 2-iodopropionic acid esters are added to the reaction mixtures.

6. Process according to Claims 1 to 5, characterised in that the reaction is carried out in technically anhydrous aprotic polar solvents or in solvent mixtures containing technically anhydrous aprotic polar solvents and water of reaction which has possibly been formed during the reaction is eliminated from the batch.

7. Process according to Claims 1 to 6, characterised in that the solvent and excess 2-chloropropionic acid ester of the formula II and 2-iodopropionic acid ester which has been formed are distilled off after the reaction, and the distillate is re-employed in the following batch.

**Revendications**

1. Procédé pour préparer des dérivés d'acides benzimidazolyloxy-, benzoxazolyloxy- et benzothiazolyloxyphénoxy-propioniques qui répondent à la formule I :

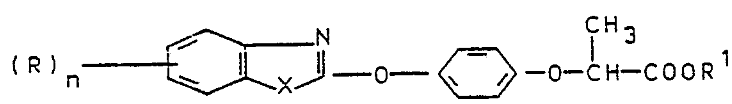 (I)

dans laquelle

X représente l'oxygène, le soufre ou un radical >N-Alkyl dont l'allkyle contient de 1 à 4 atomes de carbone,

R représente un halogène ou un radical $-CF_3$,

n désigne un nombre de 0 à 3 et

$R^1$ représente un alkyle en $C_1$-$C_{12}$ qui peut porter un alcoxy en $C_1$-$C_6$, un alkylthio en $C_1$-$C_6$, un alcoxy ($C_1$-$C_6$)-alcoxy ($C_2$-$C_4$) ou un méthoxy-éthoxy-éthoxy, ou représente un cycloalkyle en $C_5$ ou $C_6$ ou un alcoxycarbonylalkyle dont l'alcoxy contient de 1 à 4 atomes de carbone et l'alkyle 1 ou 2, par réaction de composés répondant à la formule II.

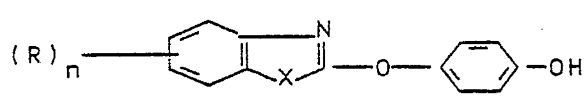 (II)

dans laquelle X, R et n ont les significations indiquées ci-dessus, avec des composés répondant à la formule III :

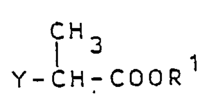 (III)

dans laquelle Y représente le chlore et $R^1$ a la signification précédemment donnée, procédé caractérisé en ce qu'on ajoute aux mélanges réactionnels, à des températures réactionnelles comprises entre 50 et 250 °C, des quantités catalytiques d'iodures métalliques et/ou d'esters de l'acide iodo-2 propionique de formule III, pour lesquels Y représente I, ainsi que des bases en des quantités au moins stœchiométriques.

2. Procédé selon la revendication 1 caractérisé en ce qu'on fait réagir un composé répondant à la formule IV :

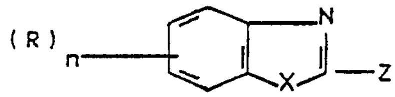

(IV)

dans laquelle R et n ont les significations précédemment données et Z représente un halogène, ou un autre substituant éliminable, avec l'hydroquinone en présence d'une base, puis, sans effectuer d'isolement, avec un ester de l'acide chloro-2 propionique répondant à la formule III :

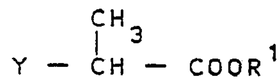

(III)

dans laquelle Y représente le chlore et $R^1$ a la signification indiquée ci-dessus, en présence de quantités catalytiques d'un iodure métallique et/ou d'un ester de l'acide iodo-2 propionique de formule III (Y représente alors l'iode), ou en ce qu'on fait réagir un composé de formule IV avec l'hydroquinone et avec un ester de l'acide chloro-2 propionique de formule III (Y représente alors le chlore), en présence d'une base et en présence de quantités catalytiques d'un iodure métallique et/ou d'un ester de l'acide iodo-2 propionique de formule III (Y représente alors l'iode).

3. Procédé selon la revendication 1 caractérisé en ce qu'on fait réagir les corps de départ de formules II et III dans un rapport molaire compris entre 1 : 1 et 1 : 5.

4. Procédé selon la revendication 2 caractérisé en ce que le rapport molaire composé de formule IV/hydroquinone/ester chloro-2 propionique de formule III (Y = Cl), au cours de la réaction, est compris entre 1 : 1 (1 à 1,5) : (1,3 à 5).

5. Procédé selon l'une quelconque des revendications 1 à 4, caractérisé en ce qu'on ajoute aux mélanges réactionnels de 0,1 à 25 % en moles d'iodures métalliques et/ou d'ester de l'acide iodo-2 propionique par rapport au composé de formule II ou IV.

6. Procédé selon l'une quelconque des revendications 1 à 5, caractérisé en ce qu'on effectue la réaction dans des solvants polaires aprotiques techniquement anhydres ou dans des mélanges de solvants contenant des solvants polaires aprotiques techniquement anhydres, et, au cours de la réaction, on élimine du mélange l'eau éventuellement engendrée par la réaction.

7. Procédé selon l'une quelconque des revendications 1 à 6, caractérisé en ce que, une fois la réaction exécutée, on chasse par distillation le solvant et l'excès de l'ester chloro-2 propionique de formule III ainsi que l'ester de l'acide iodo-2 propionique formé, et on réutilise le distillat dans l'opération qui suit.